# EUROPEAN PATENT APPLICATION

(11) **EP 2 526 925 A1**
(43) Date of publication of application: **28.11.2012**
(21) Application number: 11167113.7
(22) Date of filing: 23.05.2011
(51) Int. Cl.: A61K 9/00, A61K 31/13, A61M 15/00, B65D 83/52

(54) **A dose adjustable oral pump spray or aerosol spray containing memantine**

(71) Applicant: Deva Holding Anonim Sirketi, 34303 Istanbul (TR)
(72) Inventor: Haas, Philipp Daniel, 34303, ISTANBUL (TR); Sivasligil, Ramazan, 34303, ISTANBUL (TR); Koc, Fikret, 34303, ISTANBUL (TR); Kandemir, Levent, 34303, ISTANBUL (TR)

(57) **Abstract**

This invention embraces a formulation of memantine or pharmaceutically acceptable salt thereof for mucosal administration wherein said formulation is delivered a device characterized by that spray or aerosol dispenser is capable to emit a predetermined quantity thanks to settings more than one for different doses.

## Description

### Technical Field

This invention embraces a formulation of memantine or pharmaceutically acceptable salt thereof for mucosal administration wherein said formulation is delivered by a device characterized by that spray or aerosol dispenser is capable to emit a predetermined quantity thanks to settings more than one for different doses.

### Background Art

EP 1765287 A (MERZ PHARMA GMBH & CO KGAA) 28.03.2007 discloses memantine dosage forms exhibit dose-proportionality.

### Summary of invention

Memantine containing liquid products on the market instruct that dose proportionality is performed through the dose titration procedure. However proper dose adjusting for memantine solution is a problem for elderly patients since product containing solution requires pumping more than one to reach proper dose. It is not possible to predetermine dose amount prior to actuation at once. Consequently most of the patients confuse of counting and they cannot adequately ingest the dose.

### Technical Problem

Memantine is currently used on the basis of dose titration procedure to protect patients from side effects. The dose of Ebixa® which includes memantine is gradually increased over the first three weeks of treatment: during the first week, the dose is 5 mg; in the second week, it is 10 mg; and during the third week, it is 15 mg. From week four onwards, the recommended maintenance dose is 20 mg once a day. To carry out dose titration procedure, Ebixa® which provides one pump actuation that contains 5 mg memantine hydrochloride is used as following:
- week 1: one downward pump
- week 2: two downward pumps
- week 3: three downward pumps
- week 4: and beyond four downward pumps

Patients have to downward pumps more than one after first week and eventually four times at the last tier to reach proper dose. It gives rise too much confusion for patients since they fall frequently into the suspicion whether proper numbers of presses are performed.

In the light of technical problem stated hereinbefore there is a need a solution for both easy ingestion and proper dose adjusting for memantine solution.

US 2003/0077227 A (HARRY A. DUGGER) 24.04.2003 discloses buccal spray composition for transmucosal administration and memantine is counted active principles amongst others. However there is no any disclosure on problems arising from dose titration procedure and solution to problem is also not disclosed.

### Solution to Problem

To be solve the technical problems explained foregoing, it is invented that memantine is in the form of spray wherein dispenser emits an adjustable and predeterminable dose of liquid upon each actuation of the dispenser. Dispensing valve provides a quantity of pressurized liquid which can be adjustably selected. Thus patients are able to predetermine volume of the liquid.

### Description of embodiments

An embodiment of the present invention provides that pharmaceutically effective amount of memantine or pharmaceutically acceptable salt is delivered to the oral mucosal surfaces via actuation of a pump spray.

The term of "pump spray" covers a device suitable for dispensing a spray or mist such as a spray container, an atomizer and the like.

Present invention employs a spray dispenser to achieve dispensing of a predetermined quantity of liquid containing memantine. Spray dispenser containing memantine provides a quantity of pressurized liquid wherein said quantity of liquid can be adjusted prior to dosing. Upon each actuation dispensing of constant amount of the liquid, as it is adjusted, is ensured.

In accordance with this invention, dosing indicators are placed on dispenser as 5 mg, 10 mg, 15 mg and 20 mg and each of doses are written. Each of indicators can be coloured to ensure patient compliance.

In this invention the term of "memantine" covers pharmaceutically acceptable bases, salts, isomers, racemates, racemic mixtures, individual diasteriomers, enantiomers.

US 5085351 B (JAMES H. MARTIN) 04.02.1991 defines an adjustable dose dispenser having a plurality of different sized metering chambers with a stem rotatable to select the chamber to be discharged was disclosed. This device requires a separate metering chamber for each volume to be dispensed. Device has recesses and they are of different sizes and provide metering chambers of a plurality of different sizes. In order to adjust the amount or volume dispensed upon each actuation of the nozzle and actuator, it is merely necessary to rotate the nozzle and actuator. Suitable indicia are provided on the cap and on a depending flange portion on the nozzle and actuator member to facilitate such selection.

Dispensers can be produced according to US 5183187 B (JAMES H. MARTIN ET.AL.) 02.02.1993 which employs an arrangement utilizing a piston to achieve dispensing of a predetermined quantity of fluid. It has a single chamber where the volume dispense from the chamber is adjustable. To achieve adjustment of the dosage, rotation of stem member causes the threads to move section up and down. Thus changing the size of chamber and, hence, the amount of liquid dispensed.

EP 1413529 B (MARTIN, JAMES H.) 28.04.2004 discloses an improved adjustable metering valve for dispensing pressurized liquids. The upper portion of the valve has a plurality of notches around the circumference and printed near each notch is a marking indicative of the dosage of liquid to be dispensed upon the depression of the actuator with the nozzle received into the associated notch.

According to this invention plurality of different sized metering chambers system or single chamber with a piston or any kind of dose adjustable dispenser can be used.

Dispenser can be suitable for pump spray or pump spray for oral administration or aerosol spray for oral administration.

In formulations of this invention, surfactant, solubilizer, micelle-creating agent, emulsifying agent, viscosity modifying agent, mixtures thereof and the like can be utilized.

Formulations of this invention may also contain sweetening, taste masking, antimicrobial or flavoring agents.

Various flavors or flavoring agents may be used to mask the bitterness of the active ingredient. Flavors are but not limited to bubble gum, cherry, fruit, honey, lemon, licorice, mint, orange, peppermint, spearmint, wintergreen, mixtures thereof and the like.

Sweeteners are but not limited to, corn syrup, dextrose, invert sugar, fructose, saccharin, aspartame, acesulfame-K, Stevia rebaudiana, sucralose, sorbitol, mannitol, zylitol, mixtures thereof and the like.

Preservatives are, but not limited to, sodium benzoate, benzoic acid, ethylenediaminetetraacetic acid, sorbic acid, benzethonium chloride, benzalkonium chloride, bronopol, butyl paraben, methyl paraben, ethylparaben, propyl paraben, thiomerosol, sodium propionate, chlorhexidine, chlorobutanol, chlorocresol, cresol, imidurea, phenol, phenylmercuric salts, potassium sorbate, propylene glycol, mixtures thereof and the like.

Ethanol and/or propylene glycol, benzyl alcohol, the parabens such as butylparaben-methylparaben, glycerol, propylene glycol, chlorobutanol, phenol, phenoxyethanol, and phenylethyl alcohol may also be used in formulations.

Viscosity modifying agents are but not limited to, polyvinyl alcohol, acacia, alginic acid, bentonite, carbomer, carboxymethylcellulose, carrageenan, cellulose, ceratonia, cetyl alcohol, chitosan, colloidal silicon dioxide, ethylcellulose, gelatin, guar gum, hydroxyethyl cellulose, hydroxyethylmethyl cellulose, hydroxypropyl cellulose, hypromellose, magnesium aluminum silicate, maltitol, maltodextrin, medium-chain triglycerides, methylcellulose, polydextrose, polyethylene oxide, povidone, propylene glycol alginate, sodium alginate, stearyl alcohol, sucrose, tragacanth, trehalose, wax, xanthan gum, mixtures thereof and the like.

Surfactants are, but not limited to, functionalized siloxanes, fluorinated and perfluorinated products such as perfluorinated alcohols, and polyoxyalkylenes such as the ethylene oxide and/or propylene oxide adducts of alkylphenols, the ethylene oxide and/or propylene oxide adducts of long chain alcohols or fatty acids, mixed ethylene oxide/propylene oxide block copolymers, sorbitan fatty acid esters, ethoxylated sorbitan fatty acid esters, polyethoxylated sorbitan fatty acid esters, ethoxylated alcohols fatty amine oxides, and glycerol esters, sorbitan monooleate, sorbitan sequioleate, sorbitan trioleate, polyoxyethylene sorbitan monooleate, sodium isostearyl-2-lactate,diblock and triblock copolymers based on polyester derivatives of fatty acids and poly[ethylene oxide], diblock and triblock copolymers based on polyisobutylene succinic anhydride and poly[ethylene oxide], reaction products of ethylene oxide and propylene oxide with ethylenediamine, alkyl polyoxyethylene sulfates, cholic acid and other bile acids (e.g., cholic acid, deoxycholic acid, glycocholic acid, taurocholic acid, glycodeoxycholic acid) and salts thereof (e.g., sodium deoxycholate, etc.), dioctyl sodium sulfosuccinate, glyceryl esters, phosphatide acid and their salts, potassium laurate, sodium alginate, sodium carboxymethylcellulose, sodium dodecylsulfate, and sodium lauryl sulphate, mixtures thereof, and the like.

Yet another object of the present invention is to provide an aerosol spray, which contains memantine, capable to emit a predetermined quantity wherein it has plurality of predetermined quantity of portions so as to adjust doses through plurality of spray settings. Aerosol spray package having more than one adjuster allows a user to change the setting so as to produce different doses. Patients are able to select desired doses pursuant to dose titration.

Dosing setting consists of four different dosage indicators as 5 mg, 10 mg, 15 mg and 20 mg. Each of indicators may also be coloured differently from each other.

Another embodiment of this invention covers pump spray formulation. Formulation for per pump spray comprises memantine or pharmaceutically acceptable salt thereof is from 1 % to 50 %, viscosity agent is from 1,5 % to 20 %, sweetener is from 1 % to 20 %, flavouring agent is from 2 % to 10%, solvent is up to 90 % by weight of formulation.

In accordance with this invention aerosol compositions contain a propellant and memantine and preferably excipient or mixtures of excipients for administration to mucosal surface.

The spray compositions of the invention are administered from a sealed and pressurized container.

Suitable propellants are, but not limited to, hydrocarbons (butane, propane, etc.), chlorofluorocarbons (CFC-II, CFC-12, etc.), hydrofluorocarbons (HFA-134a, HFA-227ea, etc.), ethers (dimethylether, diethylether, etc.), perfluorocarbons, mixtures thereof and the like.

Another embodiment of this invention covers aerosol spray formulation. Formulation for per canister comprises memantine or pharmaceutically acceptable salt thereof is from 1 % to 20 %, viscosity agent is from 0,5 % to 20 %, sweetener is from 0.5 % to 20 %, flavouring agent is from 0.1 % to % 10, solvent is up to 70 %, propellant is up to 90 % by weight of formulation.

Another object is to embrace pump or aerosol spray composition of memantine. In accordance with an embodiment, the pump or aeosol spray delivers the equivalent of about 5 mg of memantine HCl per 50 mcL actuation.

In yet another embodiment, the pump or aerosol spray delivers the equivalent of about 10 mg of memantine HCl per 100 mcL actuation.

In yet another embodiment, the pump or aerosol spray delivers the equivalent of about 15 mg of memantine HCl per 150 mcL actuation.

In yet another embodiment, the pump or aerosol spray delivers the equivalent of about 20 mg of memantine HCl per 200 mcL actuation.

Liquid composition containing a spray dispenser is typically administered to oral mucosal surface or added to other suitable liquids for oral ingestion such as, but not limited to, water, fruit juices, vegetable juices, soft drinks etc.

Solvent may be water or any other suitable material.

Sprayed memantine or pharmaceutically acceptable salt thereof may be completely dissolved or dispensed in canister or dispenser.

### Example 1: Pump Spray Formulation

As an example of pump spray formulation and manufacturing method thereof of this invention, but not limited to, is given as following.

Unit formula of Example 1, ingredients per dispenser, is listed in Table 1.

**Table 1**

| Ingredient | Function | Quantity/Dispenser | % (w/v) |
|---|---|---|---|
| Memantine HCl | Active Substance | 350 mg | 10.00 |
| Glycerine | Sweetener / Viscosity Increasing Agent | 170 mg | 4.86 |
| Sodium Saccharin | Sweetener | 135 mg | 3.86 |
| Strawberry Flavour | Flavouring Agent | 40 mg | 1.14 |
| Water | Solvent/Vehicle | q.s. 3.5 mL | q.s. 100 % |

Manufacturing method of Example 1 is as following;

A) Memantine HCl (700 g), Sodium Saccharin (270 g) and Strawberry Flavour (80 g) are added to water, dissolved and mixed for 15 minutes to assure complete dissolution.

B) Glycerine (340 g) is added to solution prepared in Step A, and mixed for 15 minutes.

C) Water is added to solution prepared in Step B to complete the volume to 7 L and mixed for 30 minutes.

D) Solution prepared in Step C is filled into PE or Type III glass bottles as 3.85 mL±5% per bottle.

### Example 2: Aerosol Spray Formulation

As an example of aerosol formulation and manufacturing method thereof of this invention, but not limited to, is given as following.

Unit formula of Example 2, ingredients per dispenser, is listed in Table 2.

**Table 2**

| Ingredien t | Function | Quantity/Dispenser | % (w/v) |
|---|---|---|---|
| Memantine HCl | Active Substance | 350 mg | 3.50 |
| Maltitol | Sweetener / Viscosity Increasing Agent | 155 mg | 1.55 |
| Sucralose | Sweetener | 115 mg | 1.15 |
| Cherry Flavour | Flavouring Agent | 45 mg | 0.45 |
| Water | Solvent/Vehicle | 3500 mg | 35.00 |
| HFA-134a | Propellant | q.s. 10 mL | q.s. 100 % |

Manufacturing method of Example 2 is as following;

A) Memantine HCl (350 g), Sucralose (115 g) and Cherry Flavour (45 g) are added to water, dissolved and mixed for 25 minutes to assure complete dissolution.

B) Maltitol (155 g) is added to solution prepared in Step A, and mixed for 30 minutes.

C) Remaining water is added to solution prepared in Step B and mixed for 30 minutes.

D) Solution prepared in Step C is mixed with propellant, HFA-134a, and filled into canisters as 11 mL±5% per canister.

## Claims

1. A liquid or aerosol formulation of memantine or pharmaceutically acceptable salt thereof for oral administration which is contained in an aerosol spray canister or pump spray dispenser **characterized by** that said canister or dispenser has plurality of spray settings for different doses.

2. Aerosol spray or pump spray, as claimed in claim 1, has more than one dose indicator as 5 mg, 10 mg, 15 mg and 20 mg pursuant to dose titration procedure.

3. Dose titration procedure, as claimed in claim 2, is performed as following: 5 mg dose indicator is used for the first week and 10 mg dose indicator is used for the second week and 15 mg dose indicator is used for third week and 20 mg dose indicator is used fourth and following weeks.

4. Each of dose indicators, as claimed in claim 2 and claim 3, are coloured different from each other.

5. Pump spray formulation, as claimed in claim 1, for per dispenser comprises memantine or pharmaceutically acceptable salt thereof is from 1 % to 50 %, viscosity agent is from 1,5 % to 20 %, sweetener is from 1 % to 20 %, flavouring agent is from 2 % 10, solvent is up to 90 % by weight of formulation.

6. Aerosol spray formulation, as claimed in claim 1, for per canister comprises memantine or pharmaceutically acceptable salt thereof is from 1 % to 20 %, viscosity agent is from 0,5 % to 20 %, sweetener is from 0.5 % to 20 %, flavouring agent is from 0.1 % to % 10, solvent is up to 70 %, propellant is up to 90 % by weight of formulation.

7. According to preceding claims, when 5 mg of dose indicator is selected and actuated the pump spray or aerosol spray delivers the 5 mg of memantine HCl or equivalent per 50 mcL actuation and when 10 mg of dose indicator is selected and actuated the pump spray or aerosol spray delivers 10 mg of memantine HCl or equivalent per 100 mcL actuation and when 15 mg of dose indicator is selected and actuated the pump spray delivers 15 mg of memantine HCl or equivalent per 150 mcL actuation and when 20 mg of dose indicator is selected and actuated the pump spray delivers 20 mg of memantine HCl or equivalent per 200 mcL actuation.
